# EUROPÄISCHE PATENTSCHRIFT

(11) **EP 3 621 567 B1**
(45) Veröffentlichungstag und Bekanntmachung des Hinweises auf die Patenterteilung: **23.11.2022**
(21) Anmeldenummer: 18793357.7
(22) Anmeldetag: 13.07.2018
(51) Int. Cl.: A61F 7/00

(54) **KOPPELBARE PORTABLE VORRICHTUNG ZUR THERMISCH-MEDIZINISCHEN BEHANDLUNG DER HAUT**
COUPLABLE AND PORTABLE DEVICE FOR THE THERMAL-MEDICAL TREATMENT OF THE SKIN
DISPOSITIF PORTATIF ET ADAPTÉ POUR ÊTRE COUPLÉ POUR LE TRAITEMENT THERMIQUE-MEDICAL DE LA PEAU

(30) Priorität: 24.07.2017 DE 102017006994
(43) Veröffentlichungstag der Anmeldung: 18.03.2020
(73) Patentinhaber: Kamedi GmbH, 76131 Karlsruhe (DE)
(72) Erfinder: REUTER, Christof, 77948 Friesheim (DE); HOTZ, Stefan, 69242 Mühlhausen (DE); MEYER, Armin, 13585 Berlin (DE); LIEDTKE, Lukas Heinrich, 34497 Korbach (DE)
(74) Vertreter: Durm Patentanwälte PartG mbB
(86) Internationale Anmeldenummer: PCT/DE2018/100644
(87) Internationale Veröffentlichungsnummer: WO 2019/020144

(56) Entgegenhaltungen:
- EP-A1- 3 578 146
- WO-A1-2015/109397
- CN-U- 201 799 014
- DE-A1- 10 134 878
- DE-A1- 19 954 424
- DE-A1-102014 105 631
- DE-A1-102016 013 448
- KR-A- 20110 092 630
- US-A1- 2013 172 829
- US-A1- 2017 172 227

## Beschreibung

Die Erfindung betrifft eine koppelbare Vorrichtung zur thermisch-medizinischen Behandlung der Haut, insbesondere im Falle von Hautirritationen oder leichten Verletzungen, wie sie beispielsweise durch Insektenstiche oder Pflanzen hervorgerufen werden können.

In diesem Zusammenhang ist aus der EP 1 231 875 B1 bereits eine Vorrichtung zur lokalen thermischen Behandlung von Insektenstichen bekannt, die dazu geeignet ist, mit einer Wärmebehandlung auf den betroffenen Hautbereich einzuwirken. Im Einzelnen ist diese Vorrichtung mit einem Heizelement ausgestattet, das als elektrische Heizplatte ausgeführt ist und von einer nicht weiter spezifizierten Spannungsquelle gespeist wird. Dabei ist die Temperatur der Heizplatte, um mögliche Verbrennungen zu vermeiden auf eine Höchsttemperatur von 50-65°, vorzugsweise von 55° bis 60°, begrenzt. Im Übrigen wird diese Temperatur nur für einen Zeitraum von wenigen Sekunden aufrechterhalten. Um dies sicherzustellen, ist das Heizelement mit einem Temperatursensor, sowie mit einer Steuereinrichtung versehen, um die entsprechende Wärmebehandlung, insbesondere die eingesetzte Temperatur und die Dauer der angesprochenen Behandlung gemäß den vorstehend erläuterten Vorgaben zu steuern. In der Praxis ist es aber einigermaßen unpraktisch, im Zusammenhang mit Outdoor-Aktivitäten eine Heizplatte mit sich zu führen.

Im Falle der vorstehend angesprochenen Hautirritationen hat es sich gezeigt, dass die Wirksamkeit der angesprochenen Wärmebehandlung unter anderem auch davon abhängt, dass die angesprochene Wärmebehandlung so schnell wie möglich durchgeführt wird. Nachdem aber die angesprochenen Hautirritationen in erster Linie durch Insektenstiche, Pflanzen, aber auch Fische, Algen oder Quallen oder sonstigen Nesseltiere verursacht wird, also häufig im so genannten Outdoor-Bereich, ist es sinnvoll, die angesprochene Wärmebehandlung so schnell wie möglich, d.h. am besten direkt vor Ort durchzuführen. Es versteht sich, dass eine möglichst kompakte Lösung das dauerhafte Mitführung und so eine zeitnahe Behandlung ermöglicht.

Im Übrigen ist aus der US 2017/0079834 A1 eine Methode zur Behandlung von trockenen Augen vorbekannt, bei der beheizbare Streifen oberhalb und unterhalb des menschlichen Auges aufgesetzt werden, die jeweils über Strom führende Kabel mit einer programmierbaren Kontrolleinheit verbunden sind, die die weitere Behandlung der angesprochenen Augenpartien steuert. Diese Kontrolleinheit ist zusätzlich mit einer tragbaren elektronischen Vorrichtung, beispielsweise einem Smartphone oder einem Tablet verbunden. Die Stromversorgung der beheizbaren Streifen erfolgt allerdings über die Kontrolleinheit, so dass in dieser Kontrolleinheit auch ein geeigneter Energiespeicher integriert sein muss.

Aus der WO 2015/ 109397 A1 ist eine tragbare Vorrichtung zur Behandlung von äußeren Beulen, also Anschwellungen des menschlichen Körpers, vorbekannt, die ebenfalls lehrt, mit beheizten Streifen oder Kompressen auf die betroffenen Körperpartien einzuwirken. Auch hier wird eine Wärmebehandlung durchgeführt, wobei auch hier von dem Benutzer erwartet wird, dass er für den Fall einer entsprechenden Verletzung eine vergleichsweise voluminöse tragbare Einrichtung mit sich führt, um im Ernstfall die angesprochenen Körperschwellungen behandeln zu können.

Schließlich ist aus der US 2017/017 2227 ein tragbares Kleidungsstück vorbekannt, insbesondere zur Bekleidung des Oberkörpers, in das so genannte Mikroröhren eingearbeitet sind, wobei jede dieser Röhren mit einem Einlass und einem Auslass versehen ist, so dass es möglich ist, erwärmte oder gekühlte Luft durch diese Mikroröhren zu führen, um dem Benutzer Kühlung oder Heizung zu gewähren. Im Übrigen ist dieses Kleidungsstück mit einem Umgebungssensor zur Erfassung der Umgebungstemperatur versehen, so dass eine selbsttätige Regelung in Abhängigkeit von der Umgebungstemperatur und den vermutlich vom Benutzer gewählten Voreinstellungen möglich ist. Hier geht es also um eine Temperierung des menschlichen Körpers, insbesondere des Oberkörpers, aber nicht um eine Behandlung von möglichen Hautirritationen.

Die US 2013/0172829 A1 betrifft eine Vorrichtung und ein Verfahren zur Behandlung trockener Augen.

Die DE 10 2014 105 631 A1 betrifft eine mobile Notrufvorrichtung sowie ein Notrufsystem.

Die DE 10 2016 013 448 A1 betrifft ein Verfahren und eine Vorrichtung zum automatischen Absetzen eines Notrufs von einem Fahrzeug.

Die KR 2011 0099230 A betrifft eine Vorrichtung zur Behandlung einer Talgdrüsen Krankheit, die in einem Mobilgerät betrieben wird.

Die CN 2017 99014U betrifft ein tragbares Hautbehandlungsgerät.

Außerdem sind bereits Geräte bekannt, die mittels konzentrierter Wärme und einer mikroelektrischen Steuerung gezielt den Juckreiz und die Schwellung von Insektenstichen lindern können (EP 1231875, CN102429762A, US000007537605B2, US000007637930B2, DE202012100037, DE202014104077U1, WO2007/082648, DE 000019954424 A1). Gemein haben alle vorstehenden Geräte, dass sie alleinstehend funktionieren und mit einem Gehäuse versehen sind, das zur Handhabung der vorbekannten Geräte bei der Wärmebehandlung auch benötigt wird. Keines der genannten Geräte ist internetfähig oder sonstig vernetzt.

Im Ergebnis erscheinen also alle vorbekannten Geräte für den sportlichen Outdoor-Einsatz, als etwa für Sportarten wie Free-Climbing oder Mountain-Biken oder auch längere Radtouren oder Wanderungen, bei denen das mitgenommene Gepäck in der Regel limitiert ist, nicht geeignet, weil sie aufgrund ihres Gewichtes und ihrer Größe vernünftigerweise nicht mitgeführt werden können. Dasselbe gilt auch für das dauerhafte Mitführen im alltäglichen Gebrauch. Ausgehend von diesem Stand der Technik liegt der Erfindung die Aufgabe zu Grunde, eine portable Vorrichtung zu schaffen, die in einfacher Weise platz- und gewichtssparend mitgeführt werden kann und in Verbindung mit den angesprochenen Hautirritationen schnell eine erste Linderung oder sogar eine vollständige Abhilfe schafft.

Die Lösung dieser Aufgabe gelingt mittels einer portablen Vorrichtung zur thermisch-medizinischen Behandlung der Haut und einem System gemäß den unabhängigen Ansprüchen. Vorteilhafte Ausgestaltungen der Erfindung können den abhängigen Ansprüchen entnommen werden.

Die Erfindung betrifft gemäß dem geltenden Anspruch 1 eine portable Vorrichtung zur thermisch-medizinischen Behandlung der Haut, wie sie beispielsweise in Verbindung mit Insektenstichen oder anderen Hautirritationen vorgenommen werden. Dabei besteht die erfindungsgemäße Vorrichtung im Wesentlichen aus einem kleinteiligen Behandlungsaufsatz, der nur wenige Zentimeter breit und lang ist und somit beispielsweise in der Hosentasche unproblematisch mitgeführt werden kann.

Der Behandlungsaufsatz besteht im Wesentlichen aus einem Gehäuse und ist unterseitig von einer Schnittstelle zur Koppelung mit einer mobilen Datenverarbeitungseinheit ausgestattet. Über diese Schnittstelle wird der Behandlungsaufsatz mit der mobilen Datenverarbeitungseinheit sowohl datenverbunden, als auch mit elektrischer Energie versorgt. Durch die externe Energieversorgung kann der in die mobile Datenverarbeitungseinheit üblicherweise integrierte Energiespeicher zur Bestromung eines geeignet angeordneten Heizelementes eingesetzt werden. Die so beheizte Oberfläche kann durch entsprechende Handhabung der angeschlossenen Datenverarbeitungseinheit auf die zu behandelnde Hautoberfläche aufgesetzt werden, so dass diese einer Wärmebehandlung in einem bevorzugten Bereich zwischen 50° und 65° ausgesetzt ist.

Diese thermische Behandlung dient der Linderung des Juckreizes sowie einem günstigeren Heilungsverlauf, betreffend die Behandlung von Insektenstichen, Schlangengift oder Nesselstoffen, wie sie beispielsweise von Pflanzen oder Tieren, wie etwa Quallen übertragen werden. Die erfindungsgemäße Vorrichtung kann außerdem zur Behandlung von Herpes oder anderen Hauterkrankungen eingesetzt werden.

Durch die Wärmebehandlung werden etwa eingetragene Giftstoffe zersetzt und insgesamt der Heilungsprozess beschleunigt. Dabei kann über die Datenverbindung der Behandlungsverlauf auf der Datenverarbeitungseinheit angezeigt und verfolgt werden. Hierdurch wird auch das Risiko möglicher Infektionen etwa infolge von Insektenstiche oder Zeckenbissen gesenkt.

Im gekoppelten Zustand ist die Handhabung des kleinteiligen Behandlungsaufsatzes dadurch erleichtert, dass die üblicherweise großflächigere Datenverarbeitungseinheit dazu benutzt werden kann, den Behandlungsaufsatz bestimmungsgemäß auf die zu behandelnde Hautpartie aufzusetzen.

In konkreter Ausgestaltung ist in das Gehäuse des Behandlungsaufsatzes unmittelbar unterhalb der im gekoppelten Zustand von der Datenverarbeitungseinheit abgewandten Oberfläche des Behandlungsaufsatzes ein Heizelement, das in der Regel durch einen Widerstand realisiert ist, der sich bei Besteuerung erhitzt, angeordnet. Unmittelbar benachbart zu diesem Heizelement ist ein Temperatursensor angeordnet, über den die Umgebungstemperatur des Widerstandes erfasst wird.

In weiterer Ausgestaltung ist der Temperatursensor über die Schnittstelle mit der mobilen Datenverarbeitungseinheit datenverbunden, so dass über eine geeignete Software die Temperatur des Widerstandes steuerbar und/oder regelbar ist.

Dabei sind sowohl das erwähnte Heizelement, als auch der Temperatursensor unter Zwischenschaltung eines Microcontrollers mit der mobilen Datenverarbeitungseinheit über die bereits erwähnte Schnittstelle verbunden. Die Regelung der Temperatur des Widerstandes erfolgt dabei über den erwähnten Microcontroller und daher erforderlichenfalls unabhängig von der mobilen Datenverarbeitungseinheit. Dabei können das Heizelement und der Temperatursensor in einem einzigen Bauteil zusammengefasst sein.

In vorteilhafter Ausgestaltung der Erfindung dient die Schnittstelle nicht nur zur Stromversorgung des zur Wärmebehandlung eingesetzten Widerstandes, sondern auch zur Datenverbindung mit der mobilen Datenverarbeitungseinheit. Hierdurch können weitere Informationen etwa betreffend den Behandlungsverlauf an die Datenverarbeitungseinheit übermittelt und ebendort gespeichert werden. Im Übrigen können die gewonnenen Daten auch genutzt werden, um Informationen über potentielle Insektenplagen, sowie die Verbreitung von Insekten oder Krankheiten zu gewinnen.

In abermals vorteilhafter Ausgestaltung der Erfindung, kann auch der Behandlungsaufsatz selbst bereits mit einem kleinen Energiespeicher versehen sein, so dass der Behandlungsaufsatz als solcher notfalls, beispielsweise dann wenn keine Datenverarbeitungseinheit zur Verfügung steht oder die Datenverarbeitungseinheit selbst nicht funktionsfähig ist, für eine zumindest erste lindernde Behandlung einsetzbar ist. Gegebenenfalls kann unter diesen Umständen der Behandlungsaufsatz auch als Stand-Alone-Gerät eingesetzt werden.

Die im Zusammenhang mit dieser Behandlung gewonnenen Daten können beispielsweise über den Microcontroller drahtlos, also beispielsweise per Bluetooth an die Datenverarbeitungseinheit übertragen werden.

Um den universellen Einsatz des Behandlungsaufsatzes zu ermöglichen, ist die Schnittstelle mit den herkömmlichen Anschlüssen zur Verbindung mit üblichen Datenverarbeitungseinheiten versehen, also beispielsweise einem Lightning- oder USB-Anschluss. Grundsätzlich ist auch eine kabellose Verbindung, wie etwa über Bluetooth oder Induktion möglich. Somit ist der Aufsatz in Verbindung mit allen handelsüblichen mobilen Datenverarbeitungseinheiten einsetzbar.

In vorteilhafter Ausgestaltung ist die Erfindung wassergeschützt oder gar wasserdicht ausgeführt, um den Einsatz unter widrigen Umständen, also etwa bei Regen und eine hygienische Reinigung zu ermöglichen.

In abermals vorteilhafter Ausgestaltung ist die mobile Datenverarbeitungseinheit mit einer Software bestückt, die zur Bedienung und Steuerung des Behandlungsaufsatzes eingesetzt werden kann. Um die Funktionalität und die mittels der mit dem Behandlungsaufsatz durchgeführte Behandlung zu verbessern, kann beispielsweise der Nutzer individuelle Parameter, wie etwa einen Anwendungsfall, betroffene Körperregion, Alter, Empfindlichkeit oder relevante Erkrankungen in der Software hinterlegen, so dass die Behandlung unter Berücksichtigung dieser Parameter Steuer- und/oder regelbar ist.

Im Weiteren kann die Software dazu benutzt werden, etwa bei Eintritt definierter Ereignisse einen Notruf i. V. m. einer Ortsangabe abzusetzen, also beispielsweise dann, wenn nach einer definierten Zeit nach der Behandlung keine weitere Aktivität oder Bewegung des Nutzers der erfindungsgemäßen Vorrichtung feststellbar ist.

Die Erfindung wird nachstehend anhand eines Ausführungsbeispiels näher erläutert.

Es zeigen:
- Fig. 1:: einen Behandlungsaufsatz in einer Querschnittansicht,
- Fig. 2:: eine mobile Datenverarbeitungseinheit mit angeschlossenem Behandlungsaufsatz, und
- Fig. 3:: ein Blockschaltbild zur Funktion des Behandlungsaufsatzes gemäß Figur 1.

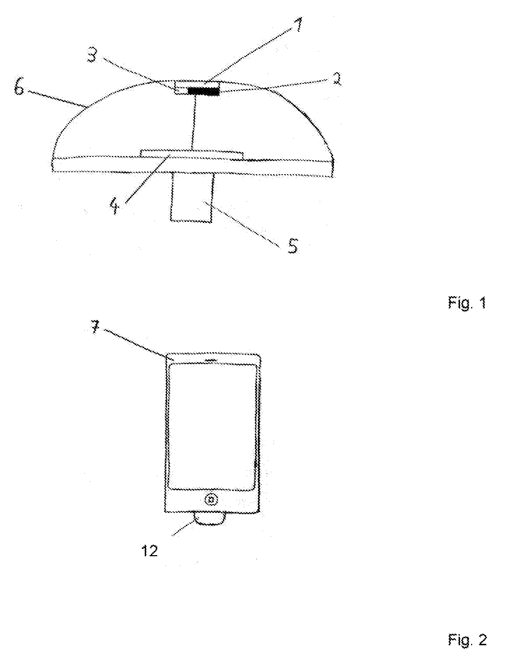

Gemäß der Darstellung in Fig. 1 umfasst der Behandlungsaufsatz 8 ein Gehäuse 6, das unterseitig von einer Schnittstelle 5 zur Verbindung mit einer in dieser Figur nicht dargestellten mobilen Datenverarbeitungseinheit 7 untergriffen ist. Innerhalb des Gehäuses 6 ist unmittelbar unterhalb der der Schnittstelle 5 abgewandten Oberfläche des Gehäuses 6 ein Heizelement 2 angeordnet, in dessen unmittelbarer Nachbarschaft zusätzlich ein Temperatursensor 3 angeordnet ist. Sobald das Heizelement 2 bestromt wird, wird dem entsprechend die der Schnittstelle 5 abgewandte Oberfläche des Gehäuses 6 erwärmt, so dass mit diesem erwärmten Abschnitt der Oberfläche des Gehäuses 6 eine Wärmebehandlung ausführbar ist. Die Stromversorgung des Heizelementes 2 erfolgt über eine elektrisch leitende Verbindung, die durch die Schnittstelle 5 unter Zwischenschaltung eines Microcontrollers 9 mit dem Heizelement 2 verbunden ist.

Fig. 2 zeigt den in Fig.1 dargestellten Behandlungsaufsatz 12 in direkter Anlage zu der mobilen Datenverarbeitungseinheit 7, in dem nämlich die Schnittstelle 5 in eine geeignete Anschlussöffnung, also beispielsweise einen Lightning-oder USB-Anschluss der mobilen Datenverarbeitungseinheit 7, vollständig eingesteckt ist, so dass der Behandlungsaufsatz 12 unmittelbar an die Datenverarbeitungseinheit 7 angeschlossen ist. Dabei erfolgt die Strom- und Datenversorgung des Heizelementes 2 und des Microcontrollers 4, wie auch des dem Heizelement zugeordneten Temperatursensors 3 über die Schnittstelle 5.

Wie ebenfalls aus dieser Figur ersichtlich, überragt der Behandlungsaufsatz 12 in der gekoppelten Stellung die Datenverarbeitungseinheit 7 nur geringfügig, so dass zur Behandlung einer Hautirritation die mobile Datenverarbeitungseinheit 7 gleichzeitig zur Handhabung des Behandlungsaufsatzes 12 dient, also vornehmlich dazu, die der mobilen Datenverarbeitungseinheit 7 abgewandte Oberfläche des Behandlungsaufsatzes 12 auf die betroffene Hautpartie zum Zwecke der Wärmebehandlung aufzusetzen.

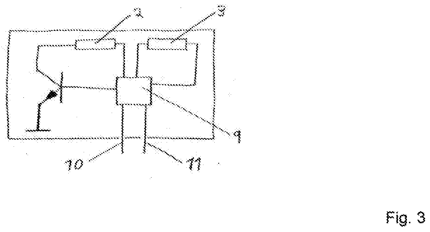

Fig. 3 zeigt ein Blockschaltbild in vereinfachter Form, betreffend die innerhalb des Behandlungsaufsatzes 12 angeordnete Schaltung. Zunächst einmal zeigt diese Schaltung, dass über die Schnittstelle 5 die in dem Gehäuse angeordnete Schaltung mit der angeschlossenen Datenverarbeitungseinheit 7 über eine Stromversorgung 10, sowie über eine Datenleitung 11 mit der Datenverarbeitungseinheit 7 verbunden ist.

In konkreter Ausgestaltung handelt es sich bei dem Heizelement 2 um einen Widerstand. Sowohl der Temperatursensor 3, wie auch das Heizelement 2 sind dabei unter Zwischenschaltung eines Microcontrollers 9 der Datenverarbeitungseinheit 7 verbunden.

In der Zeichnung nicht weiter dargestellt, aber gleichwohl möglich bzw. vorhanden ist im Rahmen der Erfindung auf der Datenverarbeitungseinheit 7 eine mobile Anwendung, also insbesondere eine App, installiert, die es zunächst einmal gestattet, den Heizvorgang zu starten und geeignete Parameter vorzugeben. In Abhängigkeit von diesen Einstellungen wird dann das Heizelement 2 aktiviert, wobei der Vorgang über den Microcontroller 9 gesteuert wird, der im Übrigen definierte Daten zur Visualisierung und Überwachung des Behandlungsvorganges an die auf der Datenverarbeitungseinheit 7 installierte mobile Anwendung übermittelt.

Der Microcontroller 9 ist üblicherweise als Bestandteil der Steuerungseinheit 4 realisiert, wobei die Steuerungseinheit 4 üblicherweise zusätzlich mit einer Transistorschaltung zur Leistungsregelung des Heizelementes versehen ist.

So kann beispielsweise das Erreichen einer vorgegebenen Temperatur oder die Beendigung des Behandlungsvorganges an die Datenverarbeitungseinheit 7 übermittelt werden.

Der ebenfalls in das Gehäuse 6 integrierte Temperatursensor besteht in vorteilhafter Ausgestaltung aus zwei thermisch nicht miteinander gekoppelten Sensoren, wobei ein Sensor die Temperatur des Heizelementes misst, während der zweite Sensor die Hauttemperatur erfasst. Aufgrund dieser Anordnung kann der Heilungsprozess genau überwacht und gesteuert werden.

Die Verbindung des Behandlungsaufsatzes 12 mit der mobilen Datenverarbeitungseinheit insbesondere in Verbindung mit einer mobilen Anwendung, die ebenfalls auf der Datenverarbeitungseinheit 7 installiert ist, ermöglicht es, den Behandlungsvorgang dadurch zu verbessern, dass die mobile Anwendung zunächst vom Anwender selbst mit Daten bestückt wird, etwa betreffend den Gesundheitszustand des Benutzers, die dann bei der Anwendung des Behandlungsaufsatzes 12 mit berücksichtigt werden können. Im Übrigen kann die Überwachung des Behandlungsprozesses durch die mobile Anwendung auch dazu genutzt werden, erforderlichenfalls einen Notruf abzusetzen, nämlich wäre in der Steuerungseinheit 4 angelegte Parameter erfüllt sind, beispielsweise wenn keine weitere Bewegung des Benutzers erfolgt oder andere Parameter eine Notsituation wahrscheinlich machen. In diesen Fällen kann über die mobile Anwendung selbsttätig ein Notruf unter Angabe des potentiellen Aufenthaltsortes des Benutzers abgesetzt werden.

Vorstehend ist somit eine portable Vorrichtung zur Behandlung von Hautirritationen oder kleineren Verletzungen, wie etwa Insektenstichen, Einwirkungen von Nessel-Pflanzen oder Nessel-Tieren beschrieben, die sehr kleinteilig ausgebildet ist und daher in einfacher Weise mitgeführt werden kann. Zur Benutzung des Behandlungsaufsatzes 12 ist lediglich ein Anschluss an eine mobile Datenverarbeitungseinheit erforderlich, die heute üblicherweise von jedermann mitgeführt wird.

### BEZUGSZEICHENLISTE

- 1: Kopplungseinheit
- 2: Heizelement
- 3: Temperatursensor
- 4: Steuerungseinheit
- 5: Schnittstelle zu einer digitalen Datenverarbeitungseinheit
- 6: Gehäuse
- 7: Mobile Datenverarbeitungseinheit
- 8: -
- 9: Microcontroller
- 10: Datenleitung
- 11: Stromversorgung
- 12: Behandlungsaufsatz

## Patentansprüche

1. Portable Vorrichtung (12) zur thermisch-medizinischen Behandlung der Haut, mit:
einer Schnittstelle (5), über die die portable Vorrichtung (12) mit einer mobilen Datenverarbeitungseinheit (7), die als Smartphone oder Tablet ausgebildet ist, koppelbar ist,
einem Gehäuse (6), das mit einem Heizelement (2) und mit einem Temperatursensor (3) versehen ist; und
einer Steuerungseinheit (4),
wobei das Heizelement (2) mit dem Temperatursensor (3) gekoppelt ist und unter Zwischenschaltung der Steuerungseinheit (4) über die Schnittstelle (5) mit der mobilen Datenverarbeitungseinheit (7) derart verbindbar ist, dass das Heizelement (2) über einen oder mehrere in die mobile Datenverarbeitungseinheit (7) integrierte Akkumulatoren derart mit Strom versorgt ist, dass das Heizelement (2) auf eine über die Steuerungseinheit (4) regelbare Temperatur erwärmbar ist;
wobei die portable Vorrichtung (12) in gekoppeltem Zustand unmittelbar an die mobile Datenverarbeitungseinheit (7) angeschlossen ist, so dass zum Zwecke der Behandlung der Haut eine der mobilen Datenverarbeitungseinheit (7) abgewandte Oberfläche des Gehäuses (6), unter der das Heizelement (2) innerhalb des Gehäuses angeordnet ist, durch entsprechende Handhabung der mobilen Datenverarbeitungseinheit (7) auf eine zu behandelnde Körperpartie aufsetzbar ist.

2. Portable Vorrichtung (12) nach Anspruch 1, **dadurch gekennzeichnet, dass** als Heizelement (2) ein sich bei Bestromung erhitzender Widerstand innerhalb des Gehäuses (6) unmittelbar unterhalb der in gekoppeltem Zustand von der mobilen Datenverarbeitungseinheit (7) abgewandten Oberfläche des Gehäuses (6) der portablen Vorrichtung (12) dem Temperatursensor (3) unmittelbar benachbart angeordnet ist, wobei sowohl der Temperatursensor (3) als auch der Widerstand über die in die Schnittstelle (5) integrierte Stromversorgung mit dem oder den Akkumulatoren der angeschlossenen mobilen Datenverarbeitungseinheit (7) verbunden sind.

3. Portable Vorrichtung (12) nach einem der vorstehenden Ansprüche, **dadurch gekennzeichnet, dass** der Temperatursensor (3) über die Schnittstelle (5) oder eine Bluetooth-Verbindung mit der mobilen Datenverarbeitungseinheit (7) datenverbindbar ist.

4. Portable Vorrichtung (12) nach einem der vorstehenden Ansprüche, **dadurch gekennzeichnet, dass** in dem Gehäuse (6) ein, vorzugsweise kleinteiliger, Energiespeicher angeordnet ist.

5. Portable Vorrichtung (12) nach einem oder mehreren der vorhergehenden Ansprüche, **dadurch gekennzeichnet, dass** sowohl das Heizelement (2) als auch der Temperatursensor (3) unter Zwischenschaltung eines Microcontrollers (9) mit der mobilen Datenverarbeitungseinheit (7) über die Schnittstelle (5) verbindbar sind.

6. Portable Vorrichtung (12) nach Anspruch 5, **dadurch gekennzeichnet, dass** die Schnittstelle sowohl elektrische Energie überträgt (11), als auch eine Datenverbindung (10) mit der in gekoppeltem Zustand angeschlossenen mobilen Datenverarbeitungseinheit (7) umfasst.

7. Portable Vorrichtung (12) nach einem der vorstehenden Ansprüche, **dadurch gekennzeichnet, dass** die portable Vorrichtung (12) in gekoppeltem Zustand über die Schnittstelle (5) mit einem der herkömmlichen Anschlüsse einer mobilen Datenverarbeitungseinheit (7), vorzugsweise einem Lightning- oder USB-Anschluss oder vergleichbaren Anschluss, etwa über Bluetooth, verbindbar ist.

8. Portable Vorrichtung (12) nach einem der vorstehenden Ansprüche, **dadurch gekennzeichnet, dass** die portable Vorrichtung (12) wassergeschützt oder wasserdicht ausgeführt ist und im Übrigen die Schnittstelle (5) mit entsprechenden Dichtungselementen zur Ausbildung einer wasserdichten oder wassergeschützten Verbindung mit der mobilen Datenverarbeitungseinheit (7) versehen sind.

9. Portable Vorrichtung nach einem der vorstehenden Ansprüche, wobei die Steuerungseinheit (4) dazu ausgebildet ist, von einer Software zur Bedienung und Steuerung der portablen Vorrichtung in der mobilen Datenverarbeitungseinheit Parameter der Wärmebehandlung vorgegeben zu bekommen.

10. System mit einer mobilen Datenverarbeitungseinheit, die als Smartphone oder Tablet ausgebildet ist, und einer portablen Vorrichtung (12) nach einem der vorstehenden Ansprüche, **dadurch gekennzeichnet, dass** die mobile Datenverarbeitungseinheit (7) über eine Software zur Bedienung und Steuerung der portablen Vorrichtung (12), insbesondere zur Steuerung der Wärmebehandlung, einsetzbar ist, indem über die Software durch entsprechende Eingaben in die mobile Datenverarbeitungseinheit (7) unter Zwischenschaltung der Steuerungseinheit (4) Parameter der Wärmebehandlung, also beispielsweise die eingesetzte Temperatur und/oder die Dauer der Wärmebehandlung, vorgebbar sind.

11. System nach Anspruch 10, **dadurch gekennzeichnet, dass** die Software mit der Geotracking-Funktion der mobilen Datenverarbeitungseinheit (7) verbunden ist, so dass entweder selbsttätig bei Eintritt in der Steuerungseinheit vorgegebener Parameter, oder aber durch eine entsprechende aktive Bedienung der Software ein Notruf in Verbindung mit einer Ortsangabe absetzbar ist.

## Claims

1. Portable device (12) for thermal-medical treatment of the skin, comprising:
an interface (5) via which the portable device (12) can be coupled to a mobile data processing unit (7), which is designed as a smartphone or tablet,
a housing (6) that is provided with a heating element (2) and a temperature sensor (3); and
a control unit (4),
wherein the heating element (2) is coupled to the temperature sensor (3) and, with the control unit (4) interconnected, can be connected via the interface (5) to the mobile data processing unit (7) in such a way that the heating element (2) is supplied with current via one or more rechargeable batteries integrated into the mobile data processing unit (7) in such a way that the heating element (2) is heatable to a temperature controllable by the control unit (4);
wherein the portable device (12) in a coupled state is attached directly to the mobile data processing unit (7), so that for the purpose of treatment of the skin a surface of the housing (6) facing away from the mobile data processing unit (7), under which the heating element (2) is arranged within the housing, can be placed by corresponding handling of the mobile data processing unit (7) on a body part to be treated.

2. Portable device (12) according to claim 1, **characterized in that**, as a heating element (2), a resistor which heats up upon energizing is arranged within the housing (6) directly adjacent to the temperature sensor (3) directly below the surface of the housing (6) of the portable device (12) facing away from the mobile data processing unit (7) in the coupled state, wherein both the temperature sensor (3) and the resistor are connected via the power supply integrated into the interface (5) to the rechargeable battery or batteries of the connected mobile data processing unit (7).

3. Portable device (12) according to any one of the preceding claims, **characterized in that** the temperature sensor (3) can have a data connection via the interface (5) or via a Bluetooth connection to the mobile data processing unit (7).

4. Portable device (12) according to any one of the preceding claims, **characterized in that** an energy accumulator, preferably a small-format energy accumulator, is arranged in the housing (6).

5. Portable device (12) according to one or more of the preceding claims, **characterized in that** both the heating element (2) and the temperature sensor (3) can be connected to the mobile data processing unit (7) via the interface (5) with a microcontroller (9) interconnected.

6. Portable device (12) according to claim 5, **characterized in that** the interface both transmits (11) electrical energy and comprises a data connection (10) to the mobile data processing unit (7) attached in the coupled state.

7. Portable device (12) according to any of the preceding claims, **characterized in that** the portable device (12) can be connected in the coupled state via the interface (5) to one of the conventional connectors of a mobile data processing unit (7), preferably a lightning or USB connector, or a comparable connector, for example via Bluetooth.

8. Portable device (12) according to any one of the preceding claims, **characterized in that** the portable device (12) is embodied as water-resistant or watertight and moreover the interface (5) is provided with corresponding seal elements to form a watertight or water-resistant connection to the mobile data processing unit (7).

9. Portable device according to any one of the preceding claims, wherein the control unit (4) is designed to be provided with parameters of the heat treatment by a software in the mobile data processing unit for operating and controlling the portable device.

10. System with a mobile data processing unit designed as a smartphone or tablet, and a portable device (12) according to any one of the preceding claims, **characterized in that** the mobile data processing unit (7) is usable via a software for operating and controlling the portable device (12), in particular for controlling the heat treatment, by parameters of the heat treatment, for example the utilized temperature and/or the duration of the heat treatment, being pre-determinable via the software by corresponding inputs into the mobile data processing unit (7) with the control unit (4) interconnected.

11. System according to claim 10, **characterized in that** the software is connected to the geo-tracking function of the mobile data processing unit (7), so that an emergency call in conjunction with a location specification can be placed either automatically upon occurrence of parameters predetermined in the control unit, or by a corresponding active operation of the software.

## Revendications

1. Dispositif portatif (12) pour le traitement thermique-médical de la peau, comprenant :
une interface (5) par laquelle le dispositif portatif (12) peut être couplé à une unité de traitement de données mobile (7) qui est conçue en tant que smartphone ou tablette,
un boîtier (6) qui est doté d'un élément chauffant (2) et d'un capteur de température (3) ; et
une unité de commande (4),
dans lequel l'élément chauffant (2) est couplé au capteur de température (3) et peut être relié à l'unité de traitement de données mobile (7) par le montage intermédiaire de l'unité de commande (4) par le biais de l'interface (5) de telle façon que l'élément chauffant (2) est alimenté en courant électrique par un ou plusieurs accumulateur(s) intégré(s) dans l'unité de traitement de données mobile (7) de telle façon que l'élément chauffant (2) peut être chauffé à une température réglable par le biais de l'unité de commande (4) ;
dans lequel le dispositif portatif (12), à l'état couplé, est raccordé directement à l'unité de traitement de données (7), de telle façon que pour le traitement de la peau, une surface du boîtier (6) détournée de l'unité de traitement de données (7) sous laquelle l'élément chauffant (2) est disposé à l'intérieur du boîtier peut être placée sur une partie de corps à traiter par une manipulation correspondante de l'unité de traitement de données mobile (7).

2. Dispositif portatif (12) selon la revendication 1, **caractérisé en ce qu'**en tant qu'élément chauffant (2), une résistance chauffant par une alimentation électrique est disposée à l'intérieur du boîtier (6) directement sous la surface du boîtier (6) du dispositif portatif (12) détournée de l'unité de traitement de données mobile (7), dans le voisinage direct du capteur de température (3), dans lequel tant le capteur de température (3) que la résistance sont reliés avec le(s) accumulateur(s) de l'unité de traitement de données mobile (7) raccordée par le biais de l'alimentation électrique intégrée dans l'interface (5).

3. Dispositif portatif (12) selon l'une des revendications précédentes, **caractérisé en ce que** le capteur de température (3) peut être en liaison de données avec l'unité de traitement de données mobile (7) par le biais de l'interface (5) ou d'une liaison Bluetooth.

4. Dispositif portatif (12) selon l'une des revendications précédentes, **caractérisé en ce qu'**un accumulateur d'énergie, de préférence formé de petites pièces, est disposé dans le boîtier (6).

5. Dispositif portatif (12) selon l'une ou plusieurs des revendications précédentes, **caractérisé en ce que** tant l'élément chauffant (2) que le capteur de température (3) peuvent être reliés à l'unité de traitement de données mobile (7) par le biais de l'interface (5) par le montage intermédiaire d'une micro-commande (9).

6. Dispositif portatif (12) selon la revendication 5, **caractérisé en ce que** l'interface transmet de l'énergie électrique (11) et comprend également une liaison de données (10) avec l'unité de traitement de données mobile (7) raccordée, dans l'état couplé.

7. Dispositif portatif (12) selon l'une des revendications précédentes, **caractérisé en ce que** le dispositif portatif (12) peut être relié à l'état couplé, par le biais de l'interface (5), à une des connexions courantes d'une unité de traitement de données mobile (7), de préférence une connexion Lightning ou USB ou une connexion comparable, par exemple par Bluetooth.

8. Dispositif portatif (12) selon l'une des revendications précédentes, **caractérisé en ce que** le dispositif portatif (12) est conçu protégé de l'eau ou étanche à l'eau, et en plus l'interface (5), est dotée d'éléments d'étanchéité correspondants pour former une liaison protégée de l'eau ou étanche à l'eau avec l'unité de traitement de données mobile (7).

9. Dispositif portatif selon l'une des revendications précédentes, dans lequel l'unité de commande (4) est conçue pour recevoir de manière prédéfinie des paramètres du traitement thermique d'un logiciel d'utilisation et de commande du dispositif portatif dans l'unité de traitement de données mobile.

10. Système comprenant une unité de traitement de données mobile qui est conçue en tant que smartphone ou tablette, et un dispositif portatif (12) selon l'une des revendications précédentes, **caractérisé en ce que** l'unité de traitement de données mobile (7) peut être utilisée par le biais d'un logiciel d'utilisation et de commande du dispositif portatif (12), en particulier pour commander le traitement thermique, **en ce que** par le biais du logiciel par des saisies correspondantes dans l'unité de traitement de données mobile (7) par le montage intermédiaire de l'unité de commande (4), des paramètres du traitement thermique peuvent être prédéfinis, par exemple la température employée et/ou la durée du traitement thermique.

11. Système selon la revendication 10, **caractérisé en ce que** le logiciel est relié à la fonction de géolocalisation de l'unité de traitement de données mobile (7) de façon à ce qu'un appel d'urgence en lien avec une indication de lieu puisse être automatiquement placé soit automatiquement à l'arrivée de paramètres prédéfinis dans l'unité de commande, soit par une commande active correspondante du logiciel.
